# EUROPEAN PATENT APPLICATION

(11) **EP 4 768 007 A1**
(43) Date of publication of application: **01.07.2026**
(21) Application number: 25153834.4
(22) Date of filing: 24.01.2025
(51) Int. Cl.: A61H 19/00, A63B 23/20, A63B 24/00, A61M 3/02

(54) **VAGINAL FUNCTION EXERCISER**

(30) Priority: 31.12.2024 CN 202411987039
(71) Applicant: Suzhou Dongquan Biotechnology Co., Ltd., Suzhou, Jiangsu 215127 (CN)
(72) Inventor: ZHU, Binhui, Suzhou, 215127 (CN); WEN, Bin, Suzhou, 215127 (CN); HAN, Rong, Suzhou, 215127 (CN)
(74) Representative: Patent 42

(57) **Abstract**

The present invention belongs to the technical field of medical devices, and in particular, relates to a vaginal function exerciser, including: an inserting end, an irrigating pipeline being arranged at a front end of the inserting end, and an expansion unit being arranged at a middle part of the inserting end; and a host, a first port of the host communicating with the expansion unit through a second hose, a second port of the host communicating with the irrigating pipeline through a first hose. A camera is arranged at the front end of the inserting end, and the camera is electrically connected to a third port of the host through a power supply and signal line. The vaginal function exerciser achieves multiple functions of vaginal exercise and irrigation, so that a patient can perform vaginal irrigation, drug administration and dilation under the guidance of a doctor.

## Description

### TECHNICAL FIELD

The present invention belongs to the technical field of medical devices, and in particular, relates to a vaginal function exerciser.

### BACKGROUND

The main treatment for gynecological cervical cancer, endometrial cancer and vaginal cancer is radiotherapy, which includes external irradiation and intravaginal brachytherapy. Most patients suffering from gynecological tumors such as cervical cancer, endometrial cancer and vaginal cancer need brachytherapy, which is a radiotherapy technology that directly applies radiation to the corresponding parts of the vagina according to the lesion site. However, while good local control is achieved, it is easy to cause atrophy, adhesion and stenosis of vaginal mucosa, especially for patients with good prognosis, which will seriously affect the subsequent life quality, especially for the sexual life quality. Therefore, it is necessary to use a vaginal syringe and a vaginal dilator for a long time within 1 to 2 years after brachytherapy to implement vaginal rehabilitation so as to reduce the influence of late vaginal toxic reaction.

According to the practice of diagnosis and treatment, vaginal rehabilitation is required after vaginal radiotherapy or other gynecological disease treatment. In the process of vaginal rehabilitation, the correct vaginal dilatation operation directly affects the rehabilitation effect, including the control of the dilatation position, the dilatation amount and the expansion process. However, because it involves gynecological treatment, patients are willing to complete the vaginal rehabilitation operation by themselves, but the existing rehabilitation instruments make it difficult for patients to accurately implement the vaginal rehabilitation operation.

Although the existing vaginal syringe can allow patients and their families to put it into the vagina for irrigation, it does not have the dilatation function. However, the existing commonly used vaginal dilator is a vaginal speculum for gynecological examination. This kind of vaginal dilator requires high medical literacy of operators, and it is difficult for ordinary patients and their families to carry out it at home, which is inconvenient to operate and difficult to implement vaginal rehabilitation exercises. Therefore, it is urgent to solve the problem with a vaginal function exerciser.

### SUMMARY

An objective of the present invention is to provide a vaginal function exerciser, so as to solve the above problem.

To achieve the above objective, the present invention provides the following technical solution:
a vaginal function exerciser includes:
an inserting end, an irrigating pipeline being arranged at a front end of the inserting end, and an expansion unit being arranged at a middle part of the inserting end; and
a host, a first port of the host communicating with the expansion unit through a second hose, the host being used for the expansion and contraction of the expansion unit, a second port of the host communicating with the irrigating pipeline through a first hose, and the host being used to pump saline for irrigation or medicine liquid for treatment.

Preferably, a camera is arranged at the front end of the inserting end, and the camera is electrically connected to a third port of the host through a power supply and a signal line.

Preferably, the inserting end includes:
a handle;
a connecting base, fixedly connected to a middle part of the handle.

Preferably, the inserting end includes an inserting rod, detachably connected to the connecting base, the expansion unit being fixedly connected to and communicating with a middle part of the inserting rod, the expansion unit and the inserting rod being arranged coaxially, and the inserting rod being of a hollow structure.

Preferably, the inserting end includes a liquid spray head, fixedly connected to a front end of the inserting rod, a liquid outlet end of the liquid spray head passing through a side wall of the front end of the inserting rod, a liquid inlet end of the liquid spray head communicating with a liquid outlet end of a communicating pipe, the communicating pipe being arranged in the inserting rod, and a liquid inlet end of the communicating pipe passing through a side wall of a tail end of the inserting rod and then communicating with the first hose.

Preferably, the inserting end includes a vent pipe, fixedly connected into the handle, one end of the vent pipe communicating with a hollow chamber in the inserting rod, the other end of the vent pipe communicating with the second hose.

Preferably, a shooting end of the camera is fixedly connected to an inner side of the front end of the inserting rod, a connecting terminal of the camera being fixedly connected to a front end of a cannula, the cannula being located in the inserting rod, a bottom end of the cannula passing through the handle, and the power supply and the signal line being electrically connected to the camera through the cannula.

Preferably, the host includes: a host box, and a power supply assembly, a control assembly, a liquid pumping assembly and an expansion assembly which are arranged in the host box.

Preferably, the power supply assembly is electrically connected to the control assembly, the liquid pumping assembly and the expansion assembly.

Preferably, the control assembly is electrically connected to the power supply and the signal line, the control assembly is electrically connected to a display screen and a control button, and the display screen and the control button are arranged outside a top panel of the host box.

Preferably, a liquid outlet end of the liquid pumping assembly communicates with the first hose.

Preferably, a connector of the expansion assembly communicates with the second hose.

Preferably, the liquid pumping assembly includes:
a bottle bracket, fixedly connected to a side of the host box, a bottle cap being placed in the bottle bracket, the bottle cap being used to be in threaded connection with a bottle, and the bottle being used to accommodate medicine liquid or normal saline; and
Preferably, the liquid pumping assembly includes a first pump body, fixedly connected into the host box, one end of the first pump body communicating with the bottle, the other end of the first pump body communicating with the first hose, and the first pump body being used to pump liquid in the bottle to the first hose.

Preferably, the first pump body is a gas pump, a gas inlet end of the first pump body communicates with the atmosphere, a gas outlet end of the first pump body communicates with one end of a connecting hose, the other end of the connecting hose communicates with an interior of the bottle through the bottle cap, a liquid outlet pipe is arranged on the bottle cap, one end of the liquid outlet pipe communicates with the interior of the bottle, and the other end of the liquid outlet pipe communicates with the first hose.

Preferably, the first pump body is a liquid pump, a liquid inlet end of the first pump body communicates with an interior of the bottle, and a liquid outlet end of the first pump body communicates with the first hose.

Preferably, the expansion assembly includes a second pump body, the second pump body communicates with an end of the second hose, and the second pump body enables the expansion unit to expand or contract.

Preferably, a heating ring is sleeved at a position where the second pump body communicates with the second hose, and the heating ring is used to heat fluid entering the inserting rod.

Preferably, the expansion unit is a multi-sphere or cylinder, and when the expansion unit is the multi-sphere, the expansion unit comprises at least one coaxial expansion ball.

Compared with the prior art, the present invention has the following advantages and beneficial effects:
during use, the inserting end is held by the patient with the hand, the inserting end is inserted into the vagina, the host is started, and the host is provided with a program, so that the expansion and contraction of the expansion unit located at the middle part of the inserting end plays a role in vaginal exercise and functional recovery. Meanwhile, the saline for irrigation or the medicine liquid for treatment is pumped by the host to the inserting end, and the vagina is subjected to irrigation and drug administration by the irrigating pipeline arranged at the front end of the inserting end, so that vaginal cleaning and drug treatment can be implemented conveniently. The vaginal function exerciser achieves double functions of vaginal exercise and irrigation, so that the patient can perform vaginal irrigation, drug administration and dilation under the guidance of a doctor, and the vaginal rehabilitation effects can be improved.

### BRIEF DESCRIPTION OF THE DRAWINGS

To describe the technical solutions in the embodiments of the present invention or in the prior art more clearly, the following briefly introduces the accompanying drawings required for describing the embodiments. Apparently, the accompanying drawings in the following description show merely some embodiments of the present invention, and those of ordinary skill in the art may still derive other drawings from these accompanying drawings without creative efforts.
FIG. 1 is a schematic structural diagram of the present invention;
FIG. 2 is a schematic structural diagram of an inserting end according to the present invention;
FIG. 3 is an internal structural diagram of a host according to the present invention;
FIG. 4 is an internal structural diagram of the present invention with a liquid storage tank and a heating ring; and
FIG. 5 is a structural diagram of a second pump body according to the present invention.

The description of reference numerals is as follow: 1. host; 2. inserting end; 3. the first hose; 4. the second hose; 5. power supply and signal line; 101. host box; 102. power supply assembly; 103. control panel; 104. the first pump body; 105. connecting hose; 106. bottle bracket; 107. bottle cap; 108. liquid outlet pipe; 109. the second pump body; 110. liquid storage tank; 111. heating ring; 201. handle; 202. connecting base; 203. cannula; 204. vent pipe; 205. communicating pipe; 206. inserting rod; 207. expansion unit; 208. camera; 209. liquid spray head; 1091. pump body shell; 1092. annular inner wall; 1093. turnplate; 1094. gear; 1095. pump body hose; 1096. extruding bearings; 1097. rack; 1098. guide block; 1099. motor; 10910. crankshaft; 10911. crankshaft connecting rod.

### DETAILED DESCRIPTION OF THE EMBODIMENTS

The technical solutions of the embodiments of the present invention are clearly and completely described below with reference to the accompanying drawings in the embodiments of the present invention. Apparently, the described embodiments are merely some rather than all of the embodiments of the present invention. All other embodiments obtained by those of ordinary skilled in the art based on the embodiments of the present invention without creative efforts shall fall within the protection scope of the present invention.

To make the objectives, features and advantages of the present invention more apparent and comprehensible, the present invention is described in more detail below with reference to the accompanying drawings and specific embodiments.

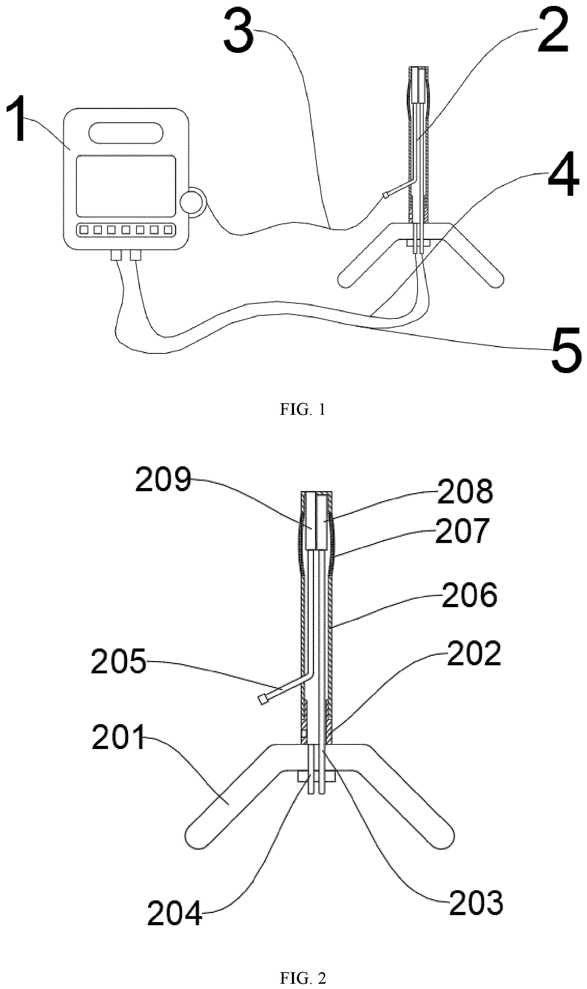

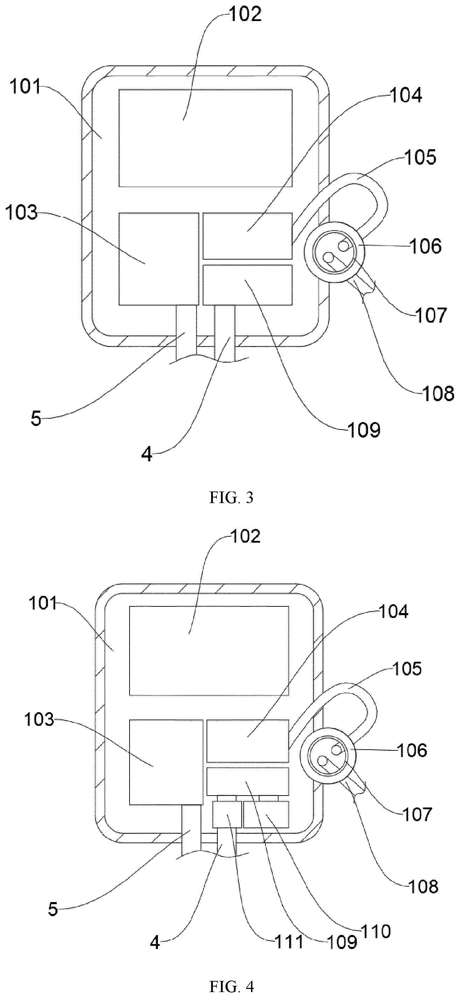

Referring to FIG. 1 to FIG. 4, the present invention discloses a vaginal function exerciser, including:
an inserting end 2, an irrigating pipeline being arranged at a front end of the inserting end 2, and an expansion unit 207 being arranged at a middle part of the inserting end 2; and
a host 1, a first port of the host 1 communicating with the expansion unit 207 through a second hose 4, the host 1 being used for the expansion and contraction of the expansion unit 207, a second port of the host 1 communicating with the irrigating pipeline through a first hose 3, and the host 1 being used to pump saline for irrigation or medicine liquid for treatment.

During use, the inserting end 2 is held by the patient with the hand, the inserting end 2 is inserted into the vagina, the host 1 is started, and the host 1 is provided with a program, so that the expansion and contraction of the expansion unit 207 located at the middle part of the inserting end 2 plays a role in vaginal exercise and functional recovery. Meanwhile, the saline for irrigation or the medicine liquid for treatment is pumped by the host 1 to the inserting end 2, and the vagina is subjected to irrigation and drug administration by the irrigating pipeline arranged at the front end of the inserting end 2, so that vaginal cleaning and drug treatment can be implemented conveniently. The vaginal function exerciser achieves double functions of vaginal exercise and irrigation, so that the patient can perform vaginal irrigation, drug administration and dilation under the guidance of a doctor, and the vaginal rehabilitation effect can be improved.

As an optional embodiment, a camera 208 is arranged at the front end of the inserting end 2, and the camera 208 is electrically connected to a third port of the host 1 through a power supply and signal line 5.

The camera 208 is arranged at the front end of the inserting end 2, so that a visualization function can be achieved.

As an optional embodiment, the inserting end 2 includes:
a handle 201;
a connecting base 202, fixedly connected to a middle part of the handle 201;
an inserting rod 206, detachably connected to the connecting base 202, the expansion unit 207 being fixedly connected to and communicating with a middle part of the inserting rod 206, the expansion unit 207 and the inserting rod 206 being arranged coaxially, and the inserting rod 206 being of a hollow structure;
a liquid spray head 209, fixedly connected to a front end of the inserting rod 206, a liquid outlet end of the liquid spray head 209 passing through a side wall of the front end of the inserting rod 206, a liquid inlet end of the liquid spray head 209 communicating with a liquid outlet end of a communicating pipe 205, the communicating pipe 205 being arranged in the inserting rod 206, and a liquid inlet end of the communicating pipe 205 passing through a side wall of a tail end of the inserting rod 206 and then communicating with the first hose 3; and
a vent pipe 204, fixedly connected into the handle 201, one end of the vent pipe 204 communicating with a hollow chamber in the inserting rod 206, the other end of the vent pipe 204 communicating with the second hose 4,
a shooting end of the camera 208 being fixedly connected to an inner side of the front end of the inserting rod 206, a connecting terminal of the camera 208 being fixedly connected to a front end of a cannula 203, the cannula 203 being located in the inserting rod 206, a bottom end of the cannula 203 passing through the handle 201, and the power supply and signal line 5 being electrically connected to the camera 208 through the cannula 203.

The handle 201 is convenient for the patient to hold and can adopt ergonomic design, the handle 201 is fixedly connected to a connecting base 202, the connecting base 202 is in friction fit or threaded connection with the inserting rod 206, and the inserting rod 206 is made of silica gel or a PVC material so as to prevent the vagina from being scratched, and the expansion unit 207 can be made of any material of silica gel, natural latex, TPU, TPE, synthetic latex, polyurethane and other balloons.

The inserting rod 206 is of a hollow structure, and is internally provided with a space for accommodating the communicating pipe 205, the cannula 203 and the camera 208.

As an optional embodiment, the host 1 includes: a host box 101, and a power supply assembly 102, a control assembly, a liquid pumping assembly and an expansion assembly which are arranged in the host box 101;
the power supply assembly 102 is electrically connected to the control assembly, the liquid pumping assembly and the expansion assembly;
the control assembly is electrically connected to the power supply and signal line 5, the control assembly is electrically connected to a display screen and a control button, and the display screen and the control button are arranged outside a top panel of the host box 101;
a liquid outlet end of the liquid pumping assembly communicates with the first hose 3; and
a connector of the expansion assembly communicates with the second hose 4.

The control assembly includes a control panel 103, the control panel 103 is electrically connected to the display screen, the control button and the camera 208, the control panel 103 is selected as a control panel with functions of programming and storing programs, optionally, a PLC programmable controller, the control panel 103 can set the expansion and contraction frequency, the expansion and contraction degree, the expansion and contraction time and other functions of the expansion unit 207 by controlling the expansion assembly, and the control panel 103 can set the liquid spray time, the liquid spray strength and other functions of the liquid spray head 209 by controlling the liquid pumping assembly. The control panel 103 is electrically connected to the camera 208, so that an image can be fed back to the display screen in real time.

As an optional embodiment, the liquid pumping assembly includes:
a bottle bracket 106, fixedly connected to a side of the host box 101, a bottle cap 107 being placed in the bottle bracket 106, the bottle cap 107 being used to be in threaded connection with a bottle, and the bottle being used to accommodate medicine liquid or normal saline; and
a first pump body 104, fixedly connected into the host box 101, one end of the first pump body 104 communicating with the bottle, the other end of the first pump body 104 communicating with the first hose 3, and the first pump body 104 being used to pump liquid in the bottle to the first hose 3.

As an optional embodiment, the first pump body 104 is a gas pump, a gas inlet end of the first pump body 104 communicates with the atmosphere, a gas outlet end of the first pump body 104 communicates with one end of a connecting hose 105, the other end of the connecting hose 105 communicates with an interior of the bottle through the bottle cap 107, a liquid outlet pipe 108 is arranged on the bottle cap 107, one end of the liquid outlet pipe 108 communicates with the interior of the bottle, and the other end of the liquid outlet pipe 108 communicates with the first hose 3.

Normal saline or medicine liquid is stored in the bottle, and then the bottle cap 107 is screwed into the bottle and placed upside down in the bottle bracket 106. When the first pump body 104 is the gas pump, the first pump body 104 is started, so that the air is pumped into the bottle. The air gathers at the top of the inverted bottle, the normal saline or medicine liquid is extruded to the liquid outlet pipe 108, and then the liquid is pumped to the liquid spray head 209 for irrigation or drug administration.

As an optional embodiment, the first pump body 104 is a liquid pump, a liquid inlet end of the first pump body 104 communicates with an interior of the bottle, and a liquid outlet end of the first pump body 104 communicates with the first hose 3.

When the first pump body 104 is the liquid pump, liquid in the bottle can be directly pumped to the liquid spray head 209.

As an optional embodiment, the expansion assembly includes a second pump body 109, the second pump body 109 communicates with an end of the second hose 4, and the second pump body 109 enables the expansion unit 207 to expand or contract.

As an optional embodiment, a heating ring 111 is sleeved at a position where the second pump body 109 communicates with the second hose 4, and the heating ring 111 is used to heat fluid entering the inserting rod 206.

As an optional embodiment, the expansion unit 207 is a multi-sphere or cylinder, and when the expansion unit 207 is the multi-sphere, the expansion unit 207 includes at least one coaxial expansion ball.

The heating temperature of the heating ring 111 does not exceed 37°C, so that damage to the inserting rod 206 and the expansion unit 207 can be avoided, and high temperature scald in the vagina can also be avoided.

When the second pump body 109 is a gas pump, the second pump body 109 pumps gas to expand the expansion unit 207, then the second pump body 109 discharges gas to contract the expansion unit 207, and so on, thereby achieving the vaginal exercise function.

The expansion amount of the expansion unit 207 can be changed by controlling the second pump body 109. A patient can select an appropriate expansion diameter according to the actual situation. Generally, the expansion diameter of the expansion unit 207 does not exceed 3.5 cm.

When the second pump body 109 is a liquid pump, liquid enters the inserting rod 206 to expand the expansion unit 207, then the second pump body 109 is reversed to return the liquid, the expansion unit 207 contracts, and so on, thereby achieving the vaginal exercise function.

When the pumped fluid is liquid, an end of the second pump body 109 away from the second hose 4 communicates with a liquid storage tank 110, and an exhaust port is formed at a side of the connecting base 202. During use, it is necessary to open the exhaust port in advance, the second pump body 109 is started to fill the inserting rod 206 with the liquid, the exhaust port is blocked after the internal air is discharged from the exhaust port, and then the second pump body 109 is started again to expand the expansion unit 207.

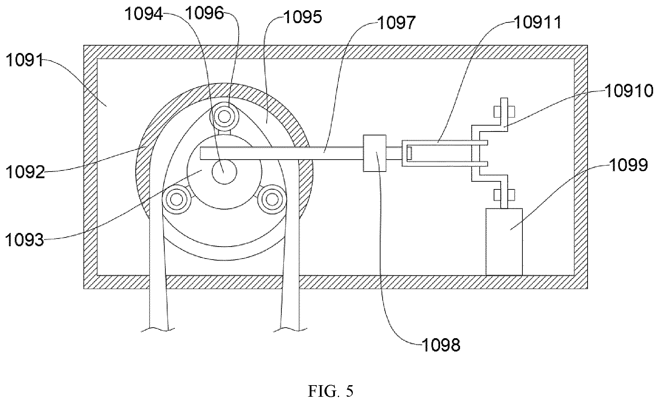

Referring to FIG. 5, as an increasable embodiment, the second pump body 109 includes:
a pump body shell 1091;
an annular inner wall 1092, fixedly connected into the pump body shell 1091;
a turnplate 1093, coaxially arranged on an inner side of the annular inner wall 1092, the turnplate 1093 being rotatably connected to the annular inner wall 1092, several extruding bearings 1096 are rotatably connected to the turnplate 1093 at equal intervals in a circumferential direction, a pump body hose 1095 being arranged between the extruding bearings 1096 and an inner wall of the annular inner wall 1092, one end of the pump body hose 1095 communicating with the second hose 4, and the other end of the pump body hose 1095 communicating with the atmosphere or the liquid storage tank 110;
a gear 1094, pivotally connected to the top of the turnplate 1093;
a rack 1097, engaged with the gear 1094, the rack 1097 being slidingly connected to a guide block 1098, and the guide block 1098 being fixedly connected into the pump body shell 1091; and
a crankshaft connecting rod 10911, one end of the crankshaft connecting rod being hinged with the rack 1097, the other end of the crankshaft connecting rod 10911 being hinged with a crankshaft 10910, the crankshaft 10910 being rotatably arranged in the pump body shell 1091, an end of the crankshaft 10910 being pivotally connected to an output shaft of a motor 1099, and a fixed end of the motor 1099 being fixedly connected into the pump body shell 1091.

During use, the motor 1099 is started, and the rack 1097 is driven by the crankshaft 10910 and the crankshaft connecting rod 10911 to reciprocate in the guide block 1098, so that the rack 1097 drives the turnplate 1093 through the gear 1094 to drive the several extruding bearings 1096 to move and extrude the pump body hose 1095, an end of the pump body hose 1095 communicates with the atmosphere or the liquid storage tank 110 the fluid is pumped into the inserting rod 206 to expand the expansion unit 207, and the rack 1097 reciprocates to change the rotating direction of the tumplate 1093, so that the pumping direction can be changed, the expansion and contraction of the expansion unit 207 can be achieved, and the expansion and contraction frequency of the expansion unit 207 can be changed by changing the rotating speed of the motor 1099.

In the description of the present invention, it should be understood that orientations or position relationships indicated by terms "longitudinal", "transverse", "upper", "lower", "front", "rear", "left", "right", "vertical", "horizontal", "top", "bottom", "inside", "outside" and the like are orientations or position relationships shown in the drawings, and these terms are merely for facilitating description of the present invention, but not for indicating or implying that the mentioned device or elements must have a specific orientation and must be established and operated in a specific orientation, and thus, these terms cannot be understood as a limitation to the present invention.

The above embodiments are only for describing the preferred mode of the present invention, and do not limit the scope of the present invention. Various modifications and improvements made by those of ordinary skill in the art without departing from the design spirit of the present invention should fall within the protection scope determined by the claims of the present invention.

## Claims

1. A vaginal function exerciser, comprising:
an inserting end (2), an irrigating pipeline being arranged at a front end of the inserting end (2), and an expansion unit (207) being arranged at a middle part of the inserting end (2); and
a host (1), a first port of the host (1) communicating with the expansion unit (207) through a second hose (4), the host (1) being used for the expansion and contraction of the expansion unit (207), a second port of the host (1) communicating with the irrigating pipeline through a first hose (3), and the host (1) being used to pump saline for irrigation or medicine liquid for treatment.

2. The vaginal function exerciser according to claim 1, **characterized in that**, a camera (208) is arranged at the front end of the inserting end (2), and the camera (208) is electrically connected to a third port of the host (1) through a power supply and a signal line (5).

3. The vaginal function exerciser according to claim 2, **characterized in that** the inserting end (2) comprises:
a handle (201);
a connecting base (202), fixedly connected to a middle part of the handle (201);
an inserting rod (206), detachably connected to the connecting base (206), the expansion unit (207) being fixedly connected to and communicating with a middle part of the inserting rod (206), the expansion unit (207) and the inserting rod (206) being arranged coaxially, and the inserting rod (206) being of a hollow structure;
a liquid spray head (209), fixedly connected to a front end of the inserting rod (206), a liquid outlet end of the liquid spray head (209) passing through a side wall of the front end of the inserting rod (206), a liquid inlet end of the liquid spray head (209) communicating with a liquid outlet end of a communicating pipe (205), the communicating pipe (205) being arranged in the inserting rod (206), and a liquid inlet end of the communicating pipe (205) passing through a side wall of a tail end of the inserting rod (206) and then communicating with the first hose (3); and
a vent pipe (204), fixedly connected into the handle (201), one end of the vent pipe (204) communicating with a hollow chamber in the inserting rod (206), the other end of the vent pipe (204) communicating with the second hose (4),
a shooting end of the camera (208) being fixedly connected to an inner side of the front end of the inserting rod (206), a connecting terminal of the camera (208) being fixedly connected to a front end of a cannula (203), the cannula (203) being located in the inserting rod (206), a bottom end of the cannula (203) passing through the handle (201), and the power supply and the signal line (5) being electrically connected to the camera (208) through the cannula (203).

4. The vaginal function exerciser according to claim 3, **characterized in that** the host (1) comprises: a host box (101), and a power supply assembly (102), a control assembly, a liquid pumping assembly and an expansion assembly which are arranged in the host box (101);
the power supply assembly (102) is electrically connected to the control assembly, the liquid pumping assembly and the expansion assembly;
the control assembly is electrically connected to the power supply and the signal line (5), the control assembly is electrically connected to a display screen and a control button, and the display screen and the control button are arranged outside a top panel of the host box (101);
a liquid outlet end of the liquid pumping assembly communicates with the first hose (3); and
a connector of the expansion assembly communicates with the second hose (4).

5. The vaginal function exerciser according to claim 4, **characterized in that** the liquid pumping assembly comprises:
a bottle bracket (106), fixedly connected to a side of the host box (101), a bottle cap (107) being placed in the bottle bracket (106), the bottle cap (107) being used to be in threaded connection with a bottle, and the bottle being used to accommodate medicine liquid or normal saline; and
a first pump body (104), fixedly connected into the host box (101), one end of the first pump body (104) communicating with the bottle, the other end of the first pump body (104) communicating with the first hose (3), and the first pump body (104) being used to pump liquid in the bottle to the first hose (3).

6. The vaginal function exerciser according to claim 5, **characterized in that**, the first pump body (104) is a gas pump, a gas inlet end of the first pump body (104) communicates with the atmosphere, a gas outlet end of the first pump body (104) communicates with one end of a connecting hose (105), the other end of the connecting hose (105) communicates with an interior of the bottle through the bottle cap (107), a liquid outlet pipe is arranged on the bottle cap (107), one end of the liquid outlet pipe (108) communicates with the interior of the bottle, and the other end of the liquid outlet pipe (108) communicates with the first hose (3).

7. The vaginal function exerciser according to claim 5, **characterized in that**, the first pump body (104) is a liquid pump, a liquid inlet end of the first pump body (104) communicates with an interior of the bottle, and a liquid outlet end of the first pump body (104) communicates with the first hose (3).

8. The vaginal function exerciser according to claim 4, **characterized in that**, the expansion assembly comprises a second pump body (109), the second pump body (109) communicates with an end of the second hose (4), and the second pump body (109) enables the expansion unit (207) to expand or contract.

9. The vaginal function exerciser according to claim 8, **characterized in that**, a heating ring (111) is sleeved at a position where the second pump body (109) communicates with the second hose (4), and the heating ring (111) is used to heat fluid entering the inserting rod (206).

10. The vaginal function exerciser according to claim 1, **characterized in that**, the expansion unit (207) is a multi-sphere or cylinder, and when the expansion unit (207) is the multi-sphere, the expansion unit (207) comprises at least one coaxial expansion ball.
